# EUROPÄISCHE PATENTSCHRIFT

(11) **EP 3 008 164 B1**
(45) Veröffentlichungstag und Bekanntmachung des Hinweises auf die Patenterteilung: **21.11.2018**
(21) Anmeldenummer: 14728219.8
(22) Anmeldetag: 06.06.2014
(51) Int. Cl.: C12M 1/107

(54) **AUFBAUVERFAHREN EINES FERMENTERS FÜR EINE BIOGASANLAGE**
CONSTRUCTION METHOD OF A FERMENTER FOR A BIOGAS PLANT
PROCÉDÉ DE MONTAGE D'UN FERMENTATEUR POUR UNE INSTALLATION DE BIOGAZ

(30) Priorität: 10.06.2013 CH 10922013
(43) Veröffentlichungstag der Anmeldung: 20.04.2016
(73) Patentinhaber: Hitachi Zosen Inova AG, 8005 Zürich (CH)
(72) Erfinder: KIENTZ, Hans-Peter, 78224 Singen (DE)
(74) Vertreter: Schaad, Balass, Menzl & Partner AG
(86) Internationale Anmeldenummer: PCT/EP2014/061841
(87) Internationale Veröffentlichungsnummer: WO 2014/198667

(56) Entgegenhaltungen:
- EP-A1- 0 794 247
- WO-A1-2009/134857
- WO-A2-2012/169983
- US-A- 2 287 198

## Beschreibung

### Technisches Gebiet

Die vorliegende Erfindung beschreibt ein Aufbauverfahren eines Fermenters mit einem Fermenterkäfig und einer Fermenterhülle aus Metall, zum Einsatz in einer Biogasanlage.

### Stand der Technik

Es ist ein Fermenter der Anmelderin für Biogasanlagen mit einem Fermenterinnenraum von etwa achtzehnhundert Kubikmetern aus einer Stahlbaukonstruktion bekannt. Dieser Fermenter weist einen Fermenterkäfig mit einer Mehrzahl von metallischen Käfigringen auf. Bei dem bekannten Herstellungsverfahren werden die Käfigringe in einem ersten Schritt vertikal auf Käfighaltevorrichtungen aufgestellt und mit Pendelstützen fixiert. Die Käfigringe bilden einen Fermenterkäfig in dessen Innenraum eine Mehrzahl von Hüllblechen angeordnet und fixiert werden. Die Hüllbleche werden mit den jeweils benachbart angeordneten Hüllblechen verschweisst, sodass eine zylindrische gasdicht verschlossene Fermenterhülle um den Fermenterkäfig angeordnet, gebildet wird.

Um einen solchen Fermenter aufzubauen, benötigt man etwa vier Monate Aufbauzeit, wobei eine hohe Werkfertigung als Vorarbeit geleistet werden muss. Das Aufstellen der Käfigringe benötigt einen grossen Portalkran, der einzelne Ringsegmente vor Ort zu Halteringen zusammenfügen kann. Ein solcher Portalkran ist nicht an jedem Aufstellort verfügbar, sodass dieser gesondert angeliefert werden muss. Ausserdem ist das Aufstellen respektive der Abbau, eines solchen Portalkrans mühsam und langwierig.

Wenn die Käfigringe auf den Käfighaltevorrichtungen fixiert sind, kann mit der Platzierung und Befestigung von vorgebogenen Hüllblechen begonnen werden. Die bislang verwendeten Hüllbleche wurden in Werkfertigung auf die Krümmung der Käfigringe abgestimmt vorgebogen und anschliessend an den Aufbauort transportiert. Neben des aufwändigen Biegens der Hüllbleche ist die Beförderung der werkgefertigten Bauteile problematisch. Ein Transport der Hüllbleche, welche etwa 8m lang und 3m breit sind, erfordert ein passendes Fahrzeug, sowie Lagervorrichtungen auf dem Fahrzeug. Je nachdem, wie weit der Anfahrtsweg zum Aufbauort ist, ist hier mit hohen Kosten zu rechnen.

Die Hüllbleche werden in den Fermenterkäfiginnenraum benachbart zueinander eingebracht und dort festgeschweisst. Dabei werden die Hüllbleche an den Innenflächen der Käfigringe fixiert und an zwischen benachbarten Hüllblechen verlaufenden Längsschweissnähten. Das Verschweissen muss sorgfältig durchgeführt werden, damit später ein gasdichter Fermenter erreicht wird. Die Hüllbleche werden dazu den gesamten Fermenterkäfig umhüllend den gesamten Fermenterkäfig auskleidend aufwändig, teilweise über Kopf verschweisst. Eine reproduzierbar gute Verschweissung ist schwierig, sehr zeitaufwändig und kann in der Regel nur von speziell ausgebildetem Personal durchgeführt werden. Eine gasdichte Verschweissung ist aber essentiell.

Nach der gasdichten Auskleidung des Fermenterkäfigs mit den Hüllblechen konnten eintragsseitige und austragsseitige Wandstrukturen am Fermenterkäfig befestigt werden. Um aber ein erforderliches Rührwerk im Fermenterkäfig zu platzieren mussten die stirnseitigen Wandstrukturen, sowie Hüllbleche im Bereich der Fermenterkäfigdecke nochmals entfernt werden, um das Rührwerk durch den Deckenbereich in den Fermenterkäfiginnenraum einzulassen. Dieser Vorgang hat den Aufbau bekannter Fermenter mit derartigen Fermenterkäfigen und Fermenterhüllen stark kompliziert und zeitlich verlängert.

Aus dem Dokument WO 2009/134857 A1 ist ein Komposter bekannt, welcher aus einer Vielzahl von Teilzylindern gebildet wird. Diese Teilzylinder bestehen wiederum aus vorgebogenen Panelen, die in einem ersten Schritt zusammengefügt werden. Erst nach dem Zusammenfügen der Teilzylinder wird der resultierende Komposter mit einer Abrollvorrichtung versehen. Die Abrollvorrichtung ist derart gelagert, dass der gesamte Komposter im Betrieb um eine Längsachse rotiert wird. Beim Aufbau des Komposters aus WO 2009/134857 A1 werden die Panele miteinander aufwendig verschweisst unter Zuhilfenahme eines zusätzlichen Schweisstisches.

Der aus Dokument WO 2009/134857 A1 bekannte Komposter hat somit den Nachteil, dass der Aufbau sehr umständlich ist. Es werden vorgebogene Panele verwendet, die an den Ort des Aufbaus geliefert werden müssen. Ein weiterer Nachteil ist, dass zusätzliche Spezialgeräte wie der Schweisstisch notwendig sind beim Aufbau.

Aus dem Dokument EP 0 794 247 A1 ist ein Fermenter bekannt, welcher mit einer abwechselnd in beide Drehrichtungen rotierenden Trommel und einer darin eingebauten Spirale, die den Innenraum in einen durchgängigen spiraligen Kanal unterteilt, versehen ist. Das zu vergärende Material durchläuft diesen Kanal nach dem Prinzip des Propfenstroms. Die Verweilzeit wird durch die Länge des Kanals und durch die Kombination aus Vorwärts- und Rückwärtsdrehungen der Trommel bestimmt. Zwischen den Wendeln der Spirale sind achsparallele Rührschaufeln angeordnet.

In der US 2 287 198 wird ein Verfahren zum Aufbau von grossen zylindrischen Tanks offenbart, bei welchem zunächst zwei voneinander beabstandete Tragringe mittels Halteseilen fixiert werden und an jedem Tragring anschliessend jeweils eine Tankhälfte aufgebaut wird. Die zwei einzelnen Tankhälften werden anschliessend mittels eines Schliessrings verbunden.

### Darstellung der Erfindung

Die vorliegende Erfindung hat sich zur Aufgabe gestellt ein Aufbauverfahren für Fermenter in Stahlbaukonstruktion zu schaffen, welches mit erheblich reduzierter Bauzeit, unter verringertem Einsatz von Spezialgeräten und weniger Werkfertigungsschritten durchführbar ist, sodass Fermenter in wesentlich kürzeren Bauzeiten als aus dem Stand der Technik bekannt, erstellt werden können.

Das Ziel ist es die Transportkosten und den Transportaufwand wesentlich zu reduzieren und Möglichkeiten zur lokalen Beschaffung von Halbzeugen zu liefern.

Die Aufgabe wird durch die Durchführung des erfindungsgemässen Verfahrens mit den Merkmalen des Patentanspruches 1 gelöst. Mit Hilfe des erfindungsgemässen Verfahrens kann ein wesentlich kostengünstigerer Aufbau derartiger Fermenter erreicht werden.

Bei der Durchführung des vorliegenden Aufbauverfahrens werden weitgehend Standardmaterial beispielsweise einfache ungebogene Stahlbleche als Hüllbleche eingesetzt werden.

### Kurze Beschreibung der Zeichnungen

Ein bevorzugtes Ausführungsbeispiel des Erfindungsgegenstandes wird nachstehend im Zusammenhang mit den anliegenden Zeichnungen beschrieben.
- Figur 1: zeigt eine perspektivische Ansicht eines fertigen betriebsbereiten Fermenters in Betriebslage abgestützt auf Stabilisierungsstützen.
- Figur 2: zeigt eine perspektivische Ansicht einer Käfighaltevorrichtung, umfassend zwei Halterungen, welche jeweils eine Abrollvorrichtung aufweisen.
- Figur 3: a) zeigt eine perspektivische Ansicht dreier Ringsegmente, welche gemäss Figur 3b) einen geschlossenen Käfigring bilden.
- Figur 4a: zeigt eine perspektivische Ansicht eines ersten Käfigringes, der auf einer zugehörigen Käfighaltevorrichtung vertikal stehend gelagert ist, während
- Figur 4b: die Verbindung zweier benachbarter Käfigringe mittels Verbundstreben und
- Figur 4c: eine perspektivische Ansicht eines fertigen Fermenterkäfigs, umfassend x Käfigringe zeigt.
- Figur 5: zeigt eine perspektivische Detailansicht einer Halterung, auf welcher ein Käfigring mittels Abrollvorrichtung rotierbar gelagert ist.
- Figuren 6: zeigen das schrittweise Einlegen und Befestigen von n Reihen von Hüllblechen in perspektivischen Ansichten, wobei nach Einlage und Befestigung jeder Reihe eine Rotation des Fermenterkäfigs erfolgt und eine geschlossene Fermenterhülle schrittweise erzeugt wird.
- Figur 7: zeigt eine perspektivische Ansicht eines Fermenterkäfigs mit angebrachter austragseitiger Wandstruktur, während
- Figur 8: eine perspektivische Ansicht des Fermenterkäfiginnenraumes mit eingebrachtem Rührwerk zeigt.
- Figur 9: zeigt den gasdicht abgeschlossenen Fermenterkäfig mit Fermenterhülle, eintragsseitiger Wandstruktur, Rührwerk und Heizsystem.
- Figuren 10: zeigen schematische Abbildungen des Fermenterkäfigs während der Montage einer Isoaltionshülle, mit mehreren Reihen von Isolationselementen, wobei der Fermenterkäfig nach Montage jeder Reihe schrittweise rotiert wird.

### Beschreibung

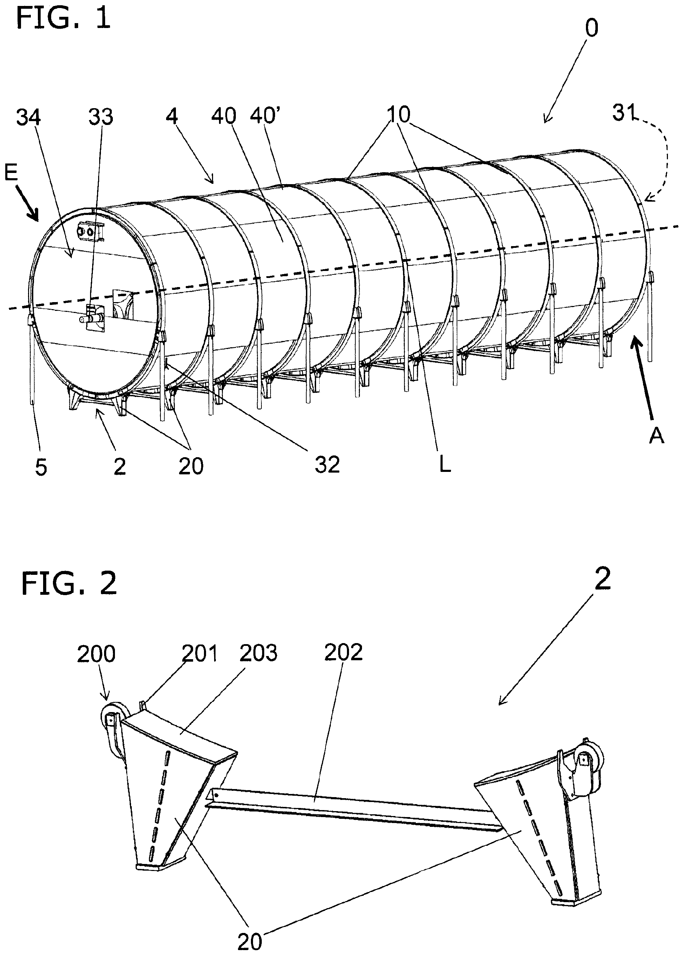

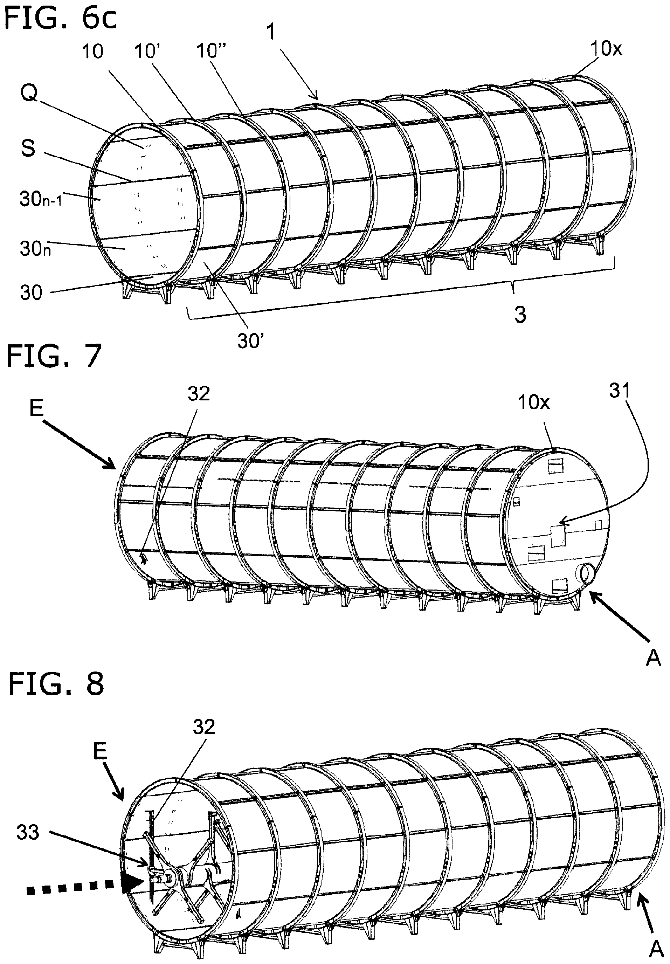

In Figur 1 ist ein fertiger gasdichter Fermenter 0 in Gebrauchslage orientiert gezeigt, der Teil einer Biogasanlage sein kann. Der Fermenter 0 erstreckt sich von einer Eintragsseite E entlang seiner Längsachse L bis zu einer Austragsseite A in etwa horizontaler Richtung. Die Eintragsseite E ist von einer eintragsseitigen Wandstruktur abgeschlossen, während die Austragsseite A von einer besser in Figur 7 gezeigten austragsseitigen Wandstruktur 31 abgeschlossen ist. Eine Mehrzahl von x Käfigringen 10 bildet einen Fermenterkäfig als Grundgerüst für den Fermenter 0. Der Fermenterkäfig wird unten im Detail beschrieben, ist aber in Figur 1 von einer Isolationshülle 4, welche mehrere Isolationselemente 40 umfasst, verdeckt. In diesem Fermenterkäfig ist ein Rührwerk 33, sowie ein Heizsystem 32 gelagert. Die Käfigringe 10 sind jeweils auf einer Käfighaltevorrichtung 2 gelagert, wobei hier jeweils zwei Halterungen 20 jeweils einen Käfigring 10 halten. Im betriebsbereiten Zustand des Fermenters 0 sind zusätzliche Stabilisierungsstützen 5 an den Käfigringen 10 in der unteren Hälfte seitlich befestigt, wodurch ein gesicherter Stand des Fermenters 0 erreichbar ist.

Um das erfindungsgemässe Aufbauverfahren durchzuführen, sind die Käfigringe 10 auf Käfighaltevorrichtungen 2, wie beispielsweise in Figur 2 dargestellt, gelagert. Die jeweils zwei Halterungen 20 sind durch eine Halterungsstrebe 202 verbunden. Jede der Halterungen 20 weist eine Abrollvorrichtung 200 und eine Auflagefläche 203 auf. Hier ist die Auflagefläche 203 auf die Krümmung der Käfigringe 10 abgestimmt. Damit die Käfigringe 10 nicht von der Auflagefläche 203 oder der Abrollvorrichtung 200 heruntergleiten, ist eine Führung 201 vorgesehen, die hier gabelartig geformt ist. Die Abrollvorrichtung 200 und die Führung 201 sind lösbar an jeder Halterung 20 befestigt. Alle Bauteile sind bevorzugt aus Stahl hergestellt.

Optional kann eine Käfighaltevorrichtung 2 mit nur einer Halterung 20 vorgesehen sein, welche entsprechend geformt sein muss, damit der Käfigring 10 stabil steht.

Um einen einfachen Transport der Bauteile für den Fermenteraufbau zu erreichen, sind die bereits eingeführten Käfigringe 10 mehrteilig ausgeführt. Hier sind drei Ringsegmente 100, 100', 100" vorgesehen, die entsprechend vorgebogen sind, um einen geschlossenen Käfigring 10 zu bilden. Die verwendeten Ringsegmente 100, 100', 100" sind Doppel-T-Träger und bestehen aus einem IPB240 Standardträger nach DIN 1025-2 und wurden kalt gebogen. An den Enden jedes Ringsegmentes 100, 100', 100" damit im Bereich der Verbindungsstellen 1000 ist jeweils eine Flanschplatte angeschweisst. Benachbarte Ringsegmente 100, 100', 100" können nach dem Zusammenfügen durch eine Mehrzahl von Schliessringbolzen miteinander bedingt lösbar verbunden werden. Andere Verbindungsmethoden sind ebenfalls möglich, bevorzugt wird aber die Verbindung mittels Schliessringbolzen, da eine einfache und schnelle Verbindung ohne Überprüfung einer Schweissnaht oder des Drehmomentes von beispielsweise Schrauben notwendig ist.

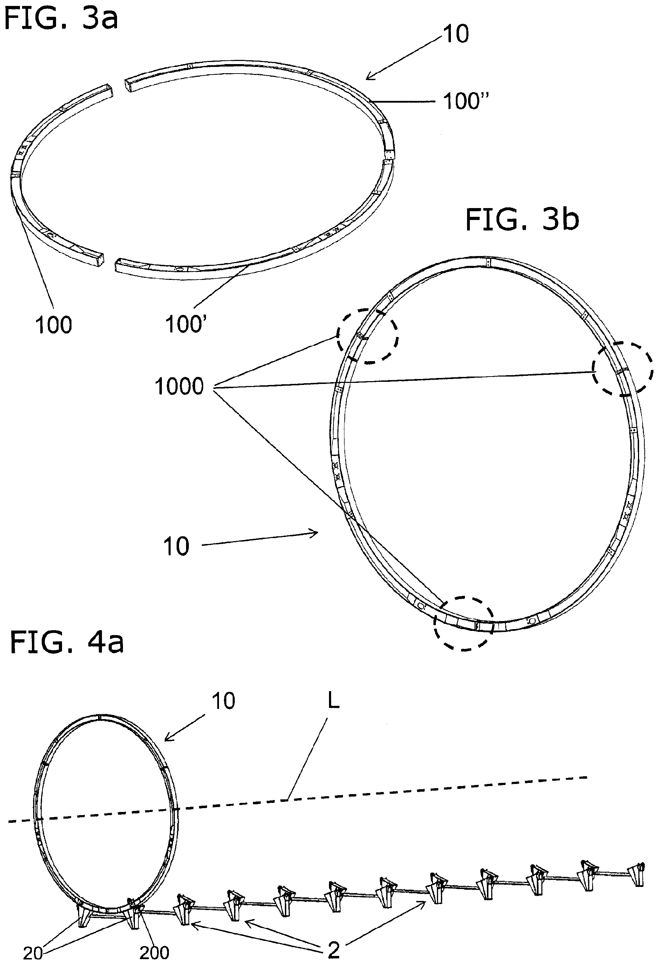

In den Figuren 4a bis 4c ist nun der schrittweise Aufbau des Fermenterkäfigs 1 dargestellt. Die Mehrzahl von Käfighaltevorrichtungen 2 ist horizontal auf einer Ebene mit festen Abständen angeordnet. Auf einer ersten Käfighaltevorrichtung 2 wird ein erster Käfigring 10 nach dem Zusammenbau aus mehreren Ringsegmenten 100, 100', 100" vertikal und damit senkrecht zur späteren Längsachse L ausgerichtet aufgestellt. Die Aussenfläche des Käfigringes 10 ist mit der Abrollvorrichtung 200, der Führung 201 und der Auflagefläche 203 jeder Halterung 20 wirkverbunden. Während des Montageprozesses rollt die Aussenfläche des Käfigringes 10 auf der Abrollvorrichtung 200 ab, sodass der Käfigring 10 um die spätere Längsachse L des Fermenters 0 rotierbar gelagert ist. Nach der fertigen Montage wird der fertige Fermenterkäfig 1 bzw. die einzelnen Käfigringe 10 auf die Auflageflächen 203 abgesenkt und damit ortsfest und drehgesichert gehalten.

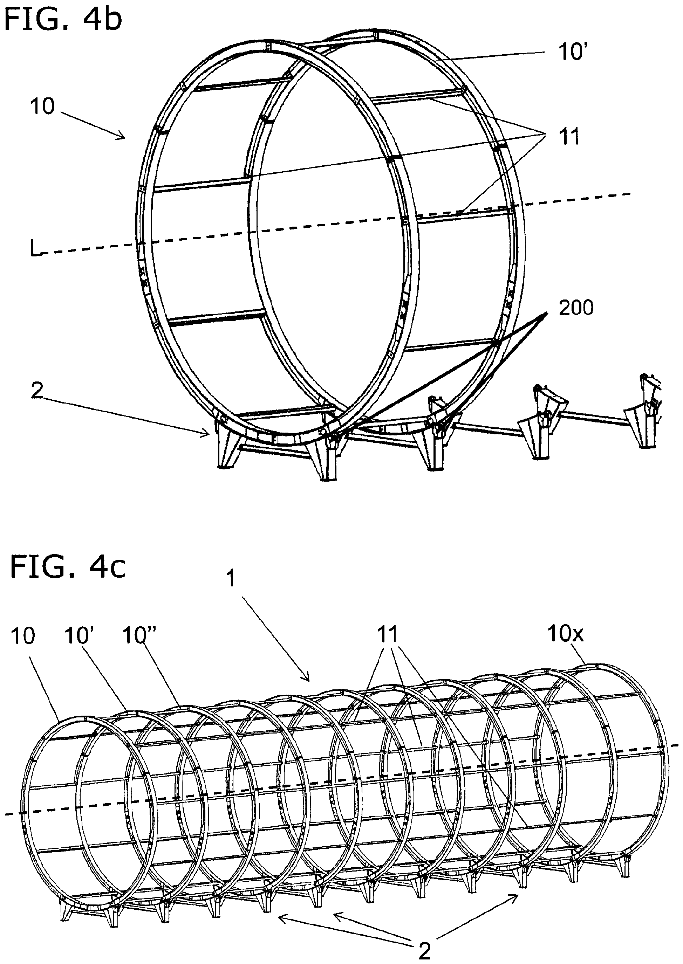

Nach dem Aufstellen eines ersten Käfigringes 10 wird ein weiterer Käfigring 10' vertikal auf eine weitere Käfighaltevorrichtung 2 wirkverbunden rotierbar aufgestellt, wie dies in Figur 4b gezeigt ist. Eine Mehrzahl von Verbundstreben 11 wird zwischen den direkt benachbarten Käfigringen 10, 10' verlaufend fixiert, sodass ein Umfallen der Käfigringe 10, 10' vermieden wird. Wie in Figur 4c gezeigt wird der Vorgang der Platzierung weiterer Käfigringe 10" bis 10x und verbindender Verbundstreben 11 solange durchgeführt, bis der Fermenterkäfig 1 die gewünschte Fermenterkäfiglänge aufweist. Es wird eine Reihenanordnung von Käfigringen 10 bis 10x und Verbindung untereinander mittels Verbundstreben 11 durchgeführt. Insgesamt wird der Fermenterkäfig 1 hier von x=11 Käfigringen 10 bis 10x gebildet. Beispielhaft sind hier jeweils neun Verbundstreben 11 entlang des Umfanges des Fermenterkäfigs 1 zwischen zwei benachbarten Käfigringen 10 bis 10x angeordnet. Die Verbundstreben 11 sind dazu lösbar, insbesondere aber unlösbar durch Anschweissen an jeweils benachbarten Käfigringe 10 bis 10x befestigt. Die Verbundstreben 11 dienen als Schweissbadsicherung für die spätere Stumpfnahtschweissung von benachbarten Hüllblechen.

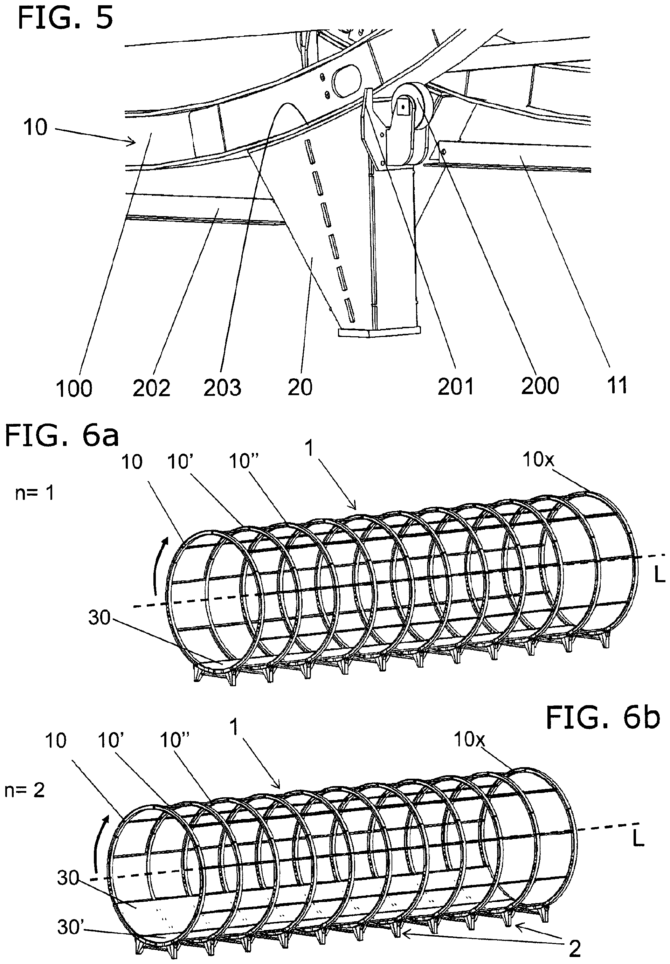

In Figur 5 ist die Auflage eines Käfigringes 10 im Detail dargestellt. Die Aussenfläche des Käfigringes 10 ist von der Auflagefläche 203 beabstandet auf der Halterung 20 bewegbar gelagert. Durch das Zusammenwirken mit der Abrollvorrichtung 200, die hier als Rolle ausgestaltet ist, kann der Käfigring 10 rotiert werden. Die Abrollvorrichtung 200 beabstandet die Aussenfläche des Käfigringes 10 geringfügig von der Auflagefläche 203, sodass eine Rotation des Käfigringes 10 ermöglicht ist. Die Führung 201 verhindert ein seitliches Abrutschen der Aussenseite des Käfigringes 10 von der Abrollvorrichtung 200 und der Auflagefläche 203.

Nach Fertigstellung des Fermenterkäfigs 1 findet die Erstellung einer Fermenterhülle 3 statt, welche aus einer Mehrzahl von n Reihen von Hüllblechen 30 bis 30n gebildet wird. Die Fermenterhülle 3 bildet eine zylindrische Mantelfläche, wobei die Fermenterhülle 3 hier innerhalb des Fermenterkäfigs 1 liegt. In einer ersten Rotationsposition des Fermenterkäfigs 1 werden in einer ersten Reihe Hüllbleche 30 in Längsrichtung also parallel zur Längsachse L in den Innenraum des Fermenterkäfigs 1 eingebracht. Durch das Eigengewicht der Hüllbleche 30 werden diese vorgebogen. Es muss aber ein aktives Biegen der ersten Hüllbleche 30 entsprechend des Verlaufs der Innenflächen der Käfigringe 10 erfolgen, wozu eine Montagehilfe eingesetzt wird. Gute Ergebnisse konnten mit Hüllbelchen 30 erreicht werden, deren Dicke kleiner gleich acht Millimeter war. Dadurch ist eine Vorbiegung erreichbar und das aktive Biegen vor Ort kann leichter durchgeführt werden. Sobald die gewünschte Krümmung erreicht ist, erfolgt eine unlösbare Befestigung der ersten Hüllbleche 30 an den zum Fermenterkäfiginneren weisenden Innenflächen der Käfigringe 10 bis 10x, bevorzugt mittels Schweissvorgang.

Die Hüllbleche 30 können mit der Innenfläche der Käfigringe 10 bis 10x mittels Lochschweissung verbunden werden.Die Montagehilfe kann nach der Befestigung der Hüllbleche 30 wieder gelöst werden, wobei das Hüllblech 30 seine gebogene Form beibehält und eine gewisse Vorspannung resultiert. Der Fermenterkäfig 1 wird durch die Mehrzahl der Hüllbleche 30 gasdicht ausgekleidet.

Wenn die erste Reihe Hüllbleche 30 befestigt ist, erfolgt eine Rotation des Fermenterkäfigs 1, was durch einen Pfeil in Figur 6a angedeutet ist. Anschliessend kann das Einlegen, Biegen und Befestigen einer weiteren Reihe von Hüllblechen 30' erfolgen, wie in Figur 6b gezeigt erfolgen. Die Hüllbleche 30' werden jeweils mit den Käfigringen 10 bis 10x entlang Querschweissnähten entlang des Umfanges der Käfigringe 10 bis 10x folgend verschweisst. Benachbarte Reihen von Hüllblechen 30, 30' werden entlang einer Längsschweissnaht S in Richtung der Längsachse L verlaufend miteinander gasdicht verschweisst. Dadurch, dass der Fermenterkäfig 1 rotierbar ist, kann die Längsschweissnaht S bevorzugt im unteren Bereich der Käfigringe 10 bis 10x, den Käfighaltevorrichtungen 2 zugewandt, erfolgen. Damit ist es möglich einen Schweissroboter zu verwenden, der die Längsschweissnähte S erzeugt.

Durch die Rotationsmöglichkeit des Fermenterkäfigs 1 kann der Fermenterkäfig 1 zur Vereinfachung der Schweissvorgänge vorgängig immer in eine derartige Schweissposition rotiert werden, dass die Längsschweissnaht S zwischen benachbarten Reihen von Hüllblechen 30 bis 30n in einer den Käfighaltevorrichtungen 2 zugewandten Position und damit im Bereich des jeweils tiefsten Punktes des zylindrischen Fermenterkäfigs 1 verschweisst wird. Ein Überkopfschweissen ist damit vermeidbar.

Um die Befestigung der Hüllbleche 30 bis 30n unterschiedlicher Reihen an den Käfigringen 10 bis 10x zu erleichtern, können eine Mehrzahl von Querschlitzen Q in den Hüllblechen 30 bis 30n vorgesehen sein. Diese Querschlitze Q sind derart angeordnet, dass sie bei in den Fermenterkäfig 1 eingelegten Hüllblechen 30 bis 30n jeweils auf den Innenflächen der Käfigringe 10 bis 10x zu liegen kommen. Dann kann durch eine Verschweissung in Umfangsrichtung die Befestigung der Hüllbleche 30 an den Käfigringen 10 bis 10x erfolgen.

Eine weitere Möglichkeit der Befestigung der eingelegten Hüllbleche 30 bis 30n besteht in einem Schweissvorgang der von ausserhalb des Fermenterkäfigs 1 erfolgt. Nach der Biegung der Hüllbleche 30 bis 30n wird eine Verschweissung der Hüllbleche in Querrichtung von ausserhalb des Fermenterkäfigs 1 durchgeführt.

Nach dem Anschweissen jeder Reihe von Hüllblechen 30, 30' wird der Fermenterkäfig 1 wie in Figur 6b angedeutet weiter rotiert werden. Die Rotation und das anschliessende Einlegen, Biegen und Befestigen weiterer Reihen von Hüllblechen 30 bis 30n wird solange wiederholt, bis eine Fermenterhülle 3 erreicht ist, welche den gesamten zylindrischen Fermenterkäfig 1 vollständig gasdicht auskleidet, was in Figur 6c dargestellt ist.

Nach Fertigstellung des Fermenterkäfigs 1 mit Fermenterhülle 3 kann der Fermenterkäfig 1 an der Austragsseite A mit einer austragsseitigen Wandstruktur 31 abgeschlossen werden. Diese austragsseitige Wandstruktur 31 wird an den letzten Käfigring 10x bzw. die Hüllbleche 30n angeschweisst.

Durch Nutzung der Rotationsmöglichkeit des Fermenterkäfigs 1 kann auch ein Heizsystem 32, umfassend eine Mehrzahl von Heizlanzen in das Fermenterkäfiginnere ragend eingesetzt und befestigt werden. Der Verlauf des Heizsystems 32 im Fermenterkäfiginneren ist in Figur 8 erkennbar. Die Dichte der Heizlanzen ist im Bereich Eintragsseite E grösser als im Bereich der Austragsseite A. Nach geeigneter Rotation des Fermenterkäfigs 1 um die Längsachse L, können die Heizlanzen einfach eingeführt werden.

Ebenfalls nach Fertigstellung der Fermenterhülle 3 kann ein Rührwerk 33 in das Fermenterkäfiginnere eingefügt und dort befestigt werden. Das Rührwerk 33 ist hier als Welle, an welcher eine Mehrzahl von Rührschaufeln radial wegragend angeordnet ist, ausgeführt. Das Rührwerk 33 wird von der Eintragsseite E linear in Richtung Längsachse L, wie durch den gestrichelten Pfeil angedeutet, in den Fermenterkäfig 1 eingeführt und befestigt. Anschliessend wird die Eintragsseite E des Ferementerkäfigs 1 mit einer eintragsseitigen Wandstruktur 34, welche am ersten Käfigring 10 befestigt wird, gasdicht verschlossen.

Der immernoch rotierbare Fermenterkäfig 1, ausgekleidet mit der Fermenterhülle 3, umfassend n Reihen von Hüllblechen 30 bis 30n wird anschliessend mittels schrittweiser Rotation des Fermenterkäfigs 1 um die Längsachse L rotiert und dabei mit einer Isolationshülle 4 versehen. Diese Isolationshülle 4 kann aus einer Mehrzahl von Isolationselementen 40 gebildet werden, welche flexibel oder starr und vorgeformt sind. Die Verwendung von vorgefertigten Isolationselementen 40 aus Schaumstoff oder Styropor ist möglich. Alternativ kann die Isolationshülle 4 bei der Rotation des ausgekleideten Fermenterkäfigs 1 durch das direkte Aufspritzen eines Schaumes gebildet werden.

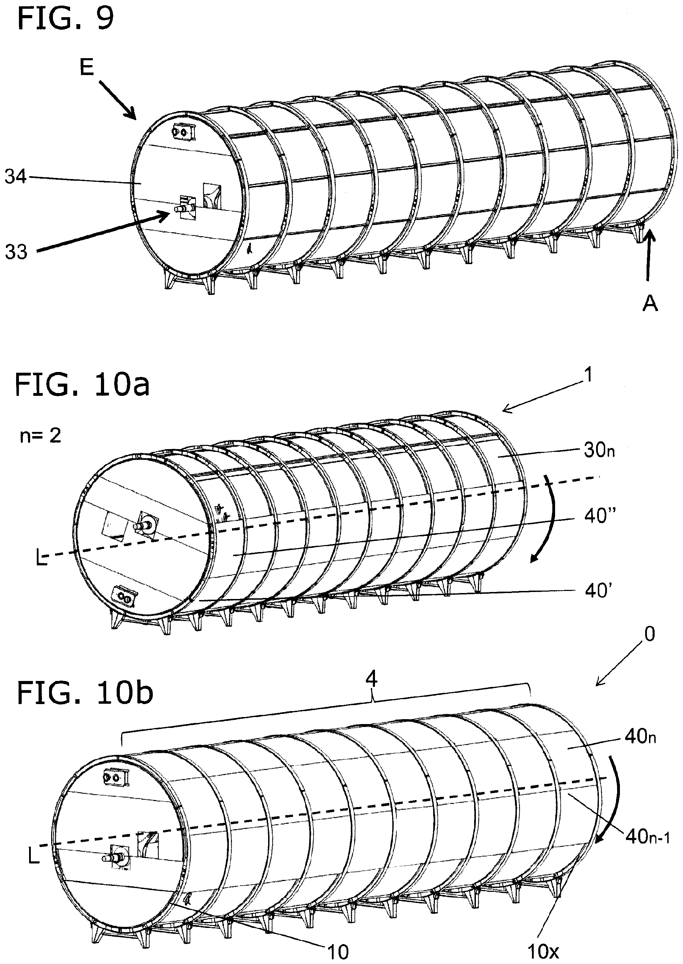

Ähnlich wie bei der Auskleidung des Fermenterkäfigs 1 mit Hüllblechen 30 bis 30n, werden Reihen von Isolationselementen 40 bis 40n auf der Fermenterhülle 3 angebracht. Nach Befestigung einer Reihe von Isolationselementen 40' auf der Aussenfläche der Fermenterhülle 3 erfolgt eine schrittweise Rotation des Fermenterkäfigs 1 um die Längsachse L, wie mittels Pfeil in Figur 10a angedeutet. Anschliessend kann eine weitere Reihe von Isolationselementen 40" aufgebracht werden. Die Isolationselemente 40 bis 40n schmiegen sich an die Aussenfläche der Fermenterhülle 3 an. Das Aufbringen der Isolationselemente 40 bis 40n und das anschliessende Rotieren des Fermenterkäfigs 1 wird solange wiederholt, bis alle Isolationselemente 40 bis 40n auf der zylindrischen Mantelfläche der Fermenterhülle 3 angeordnet und befestigt sind.

Optional könnten einzelne Isolationselemente 40 bis 40n auch bereits während der Fertigstellung der Fermenterhülle 3 nach Befestigung der Hüllbleche 30 bis 30n aufgebracht werden. Bevorzugt wird aber die Isolationshülle 4 erst nach Fertigstellung der Fermenterhülle 3 und Abschluss mit der austragsseitigen Wandstruktur 31 und der eintragsseitigen Wandstruktur 34 durch Aufbringen einzelner Reihen der Isolationselemente 40 und zwischenzeitlicher Rotation des Fermenters um die Längsachse L hergestellt.

Nach Fertigstellung der Isolationshülle 4 kann der Fermenter 0, wie in Figur 1 gezeigt in seiner Betriebsposition befestigt werden. Dazu werden die Abrollvorrichtungen 200 der Käfighaltevorrichtungen 2 entfernt und die Aussenflächen der Käfigringe 10 auf die Auflageflächen 203 abgesenkt und dort verschweisst. Der Fermenterkäfig 1 ruht dann fest auf den Käfighaltevorrichtungen 2. Zur weiteren Stabilisierung werden die Stabilisierungsstützen 5 an den Käfigringen 10 befestigt. Da die Abrollvorrichtungen 200 nach dem vollständigen Aufbau der Fermenterhülle 3 bzw. des fertigen Fermenters 0 nicht mehr benötigt werden, können diese lösbar an den Käfighaltevorrichtungen 2 befestigt sein und nach der Fertigstellung des Fermenters 0 wieder entfernt werden.

### Bezugszeichenliste

0 Fermenter
1 Fermenterkäfig
   10 Käfigringe (x Stück, 10 bis 10x)
      100, 100', 100" Ringsegment
      1000 Verbindungsstelle
   11 Verbundstreben
2 Käfighaltevorrichtung (x Stück)
   20 Halterung mit jeweils einer Abrollvorrichtung
   200 Abrollvorrichtung
   2000 Rad
   201 Führung
   202 Halterungsstrebe
   203 Auflagefläche
3 Fermenterhülle
   30 bis 30n Hüllblech (n Hüllblechreihen)
      300 Querschlitze (ausgespart zum Anschweissen)
      301 Längsnähte (zwischen direkt benachbarten Hüllblechen)
   31 austragseitige Wandstruktur
   32 Heizsystem (Heizlanzen im Randbereich des Fermenterkäfigs)
   33 Rührwerk
   34 eintragsseitige Wandstruktur
4 Isolationshülle
   40 bis 40n Isolationselemente (n Reihen Isolationselemente)
5 Stabilisierungsstützen
L Längsachse
E Eintragsseite
A Austragsseite
Q Querschlitz
S Längsschweissnaht

## Patentansprüche

1. Aufbauverfahren eines Fermenters (0) mit einem Fermenterkäfig (1) und einer Fermenterhülle (3) aus Metall, zum Einsatz in einer Biogasanlage,
**dadurch gekennzeichnet, dass**
der Fermenterkäfig (1) von einer Mehrzahl von Käfigringen (10 bis 10x) gebildet wird, wobei jeweils ein Käfigring (10 bis 10x) auf mindestens einer Käfighaltevorrichtung (2), umfassend mindestens eine Abrollvorrichtung (200) derart gelagert ist, dass der gesamte Fermenterkäfig (1) um eine Längsachse (L) rotierbar gelagert ist, und eine Mehrzahl von Verbundstreben (11) zwischen den direkt benachbarten Käfigringen (10, 10x) verlaufend fixiert wird,
wobei der Fermenterkäfig (1) durch eine Rotation um die Längsachse (L) mit einer Mehrzahl Reihen von Hüllblechen (30 bis 30n) ausgekleidet wird und die Hüllbleche (30 bis 30n) an der Mehrzahl von Käfigringen (10 bis 10x) unlösbar befestigt werden und damit die gasdichte verschlossene Fermenterhülle (3) schrittweise ausgebildet wird.

2. Aufbauverfahren eines Fermenters gemäss Anspruch 1,
**gekennzeichnet durch die Schritte:**
I) Einbringen einer ersten Reihe erster Hüllbleche (30) parallel zur Längsachse (L) in den Fermenterkäfig (1),
II) Biegen der ersten Hüllbleche (30) entsprechend des Verlaufs der Innenflächen der Käfigringe (10),
III) unlösbare Befestigung der ersten Hüllbleche (30) an den zum Fermenterkäfiginneren weisenden Innenflächen der Käfigringe (10 bis 10x) und anschliessende
IV) Rotation des Fermenterkäfigs (1) um die Längsachse (L) derart, dass eine benachbarte Reihe weiterer Hüllbleche (30' bis 30n) im Fermenterkäfig (1) neben dem vorgängig angeordneten Hüllblechen (30 bis 30n-1) platzierbar ist und
V) Wiederholung der Schritte I) bis IV) bis die n-te Reihe Hüllbleche (30 bis 30n) befestigt ist und der Fermenterkäfig (1) von der Fermenterhülle (3), umfassend n Reihen Hüllbleche (30 bis 30n), vollständig gasdicht ausgekleidet ist.

3. Aufbauverfahren eines Fermenters gemäss Anspruch 2, wobei die Herstellung des Fermenterkäfigs (1) durch
a) eine vertikale Platzierung mehrerer Käfigringe (10 bis 10x) auf Käfighaltevorrichtungen (2) mit Abrollvorrichtung (200) derart erfolgt, dass die Käfigringe (10 bis 10x) zueinander parallel angeordnet sind und mit der mindestens einen Abrollvorrichtung (200) wirkverbunden sind, sodass eine rotierbare Lagerung jedes Käfigringes (10 bis 10x) erreicht ist und
b) Befestigung mehrerer Verbundstreben (11) zwischen jeweils benachbarten Käfigringen (10 bis 10x) parallel zur Längsachse (L) des entstehenden Fermenterkäfigs (1) und Wiederholung der Schritte a) und b) solange erfolgt, bis die gewünschte Fermenterkäfiglänge erreicht ist.

4. Aufbauverfahren eines Fermenters (0) gemäss einem der vorhergehenden Ansprüche, wobei vor oder nach Fertigstellung der Fermenterhülle (3) ein Heizsystem (32) in das Fermenterkäfiginnere ragend durch Rotation des Fermenterkäfigs (1) um die Längsachse (L) angeordnet wird.

5. Aufbauverfahren eines Fermenters (0) gemäss einem der vorhergehenden Ansprüche, wobei im Anschluss an die Fertigstellung der Fermenterhülle (3) ein Rührwerk (33) in das Fermenterkäfiginnere eingefügt und dort durch Rotation des Fermenterkäfigs (1) um die Längsachse (L) befestigt wird.

6. Aufbauverfahren eines Fermenters (0) gemäss Anspruch 5, wobei das Rührwerk (33) von einer Eintragsseite (E) linear in Richtung Längsachse (L) in den Fermenterkäfig (1) eingeführt wird.

7. Aufbauverfahren eines Fermenters (0) gemäss einem der vorhergehenden Ansprüche, wobei direkt anschliessend an die Fertigstellung der Fermenterhülle (3) eine austragsseitige Wandstruktur (31) an den letzten Käfigring (10x) und eine eintragsseitige Wandstruktur (34) an den ersten Käfigring (10) gasdicht unlösbar angeschweisst wird.

8. Aufbauverfahren eines Fermenters (0) gemäss einem der vorhergehenden Ansprüche, wobei die Käfigringe (10 bis 10x) vor der Platzierung aus einer Mehrzahl von Ringsegmenten (100, 100', 100") zusammengesetzt und miteinander verbunden werden.

9. Aufbauverfahren eines Fermenters (0) gemäss Anspruch 8, wobei die Ringsegmente (100, 100', 100") an ihren Enden Flanschplatten aufweisen, welche mittels Schliessringbolzen miteinander bedingt lösbar verbunden werden.

10. Aufbauverfahren eines Fermenters (0) gemäss Anspruch 8 oder 9, wobei die Käfigringe (10 bis 10x) aus drei gleich grossen Ringsegmenten (100, 100', 100") zusammengesetzt werden.

11. Aufbauverfahren eines Fermenters (0) gemäss einem der vorhergehenden Ansprüche, wobei die Käfigringe (10 bis 10x) aus Doppel-T-Trägern gestaltet sind, welche kalt biegbar sind.

12. Aufbauverfahren eines Fermenters (0) gemäss einem der vorhergehenden Ansprüche, wobei vor der Platzierung der Käfigringe (10 bis 10x) die Mehrzahl von Käfighaltevorrichtungen (2) mit Abrollvorrichtungen (200) in einer Reihe angeordnet werden.

13. Aufbauverfahren eines Fermenters (0) gemäss einem der vorhergehenden Ansprüche, wobei die Hüllbleche (30 bis 30n) eine Dicke von acht Millimetern nicht überschreiten.

14. Aufbauverfahren eines Fermenters (0) gemäss Anspruch 1, wobei der Fermenterkäfig (1) in eine Schweissposition derart rotiert wird, dass eine Längsschweissnaht (S) zwischen benachbarten Reihen von Hüllblechen (30 bis 30n) in einer den Käfighaltevorrichtungen (2) zugewandten Position verschweisst wird.

15. Aufbauverfahren eines Fermenters (0) gemäss Anspruch 14, wobei die Verschweissung der Reihen von Hüllblechen (30 bis 30n) mit einem Schweissroboter durchgeführt wird.

16. Aufbauverfahren eines Fermenters (0) gemäss Anspruch 14, wobei eine Mehrzahl von Querschlitzen (Q) in den Hüllblechen (30 bis 30n) derart angeordnet sind, dass die Querschlitze (Q) bei in den Fermenterkäfig (1) eingelegten Hüllblechen (30 bis 30n), jeweils auf den Innenflächen der Käfigringe (10) zu liegen kommen.

17. Aufbauverfahren eines Fermenters (0) gemäss Anspruch 14, wobei nach Fixierung der Hüllbleche (30 bis 30n) an den Käfigringen (10 bis 10x), die Längsschweissnähte (S) zwischen direkt benachbarten Reihen von Hüllblechen (30 bis 30n) gasdicht verschweisst werden.

18. Aufbauverfahren eines Fermenters (0) gemäss einem der vorhergehenden Ansprüche, wobei nach dem Verschliessen der Fermenterhülle (3) eine Isolationshülle (4) durch Rotation des Fermenterkäfigs (1) um die Längsachse (L) auf der Fermenterhülle (3) aufgebracht und befestigt wird.

19. Aufbauverfahren eines Fermenters (0) gemäss Anspruch 18, wobei die Isolationshülle (4) von einer Mehrzahl starrer oder flexibler Isolationselemente (40) oder von einem Schaum gebildet wird.

## Claims

1. A construction method of a fermenter (0) with a fermenter cage (1) and a fermenter shell (3) of metal, for use in a biogas plant, **characterized in that** the fermenter cage (1) is formed by a plurality of cage rings (10 to 10x) wherein each one cage ring (10 to 10x) is supported on at least one cage-retaining device (2), comprising at least one rolling device (200), so that the entire fermenter cage (1) is mounted rotatably about a longitudinal axis (L), and a plurality of connecting braces (11) is fixed to run between the directly adjoining cage rings (10, 10'), wherein the fermenter cage (1) is lined with a plurality of rows of shell plates (30 to 30n) by means of rotation about the longitudinal axis (L), and the shell plates (30 to 30n) are permanently fastened to the plurality of cage rings (10 to 10x) and thus the gas-tight closed fermenter shell (3) is formed step by step.

2. The construction method of a fermenter as claimed in claim 1, **characterized by** the steps:
I) introducing a first row of first shell plates (30) into the fermenter cage (1) parallel to the longitudinal axis (L),
II) bending the first shell plates (30) corresponding to the path of the inner faces of the cage rings (10),
III) permanently fastening the first shell plates (30) to the inner faces of the cage rings (10 to 10x) facing the interior of the fermenter cage, and then
IV) rotating the fermenter cage (1) about the longitudinal axis (L) so that an adjacent row of further shell plates (30' to 30n) can be placed in the fermenter cage (1) next to the previously arranged shell plates (30 to 30n-1) and
V) repeating steps I) to IV) until the nth row of shell plates (30 to 30n) is fastened and the fermenter cage (1) is lined completely gas-tight by the fermenter shell (3), comprising n rows of shell plates (30 to 30n).

3. The construction method of a fermenter as claimed in claim 2, wherein the manufacture of the fermenter cage (1) is carried out by
a) vertically placing several cage rings (10 to 10x) on cage-retaining devices (2) with the rolling device (200) such that the cage rings (10 to 10x) are arranged parallel to one another and are in active connection with the at least one rolling device (200) so that a rotatable bearing of each cage ring (10 to 10x) is achieved and
b) fastening several connecting braces (11) between adjacent cage rings (10 to 10x) parallel to the longitudinal axis (L) of the resulting fermenter cage (1) and repeating the steps a) and b) until the desired fermenter cage length is reached.

4. The construction method of a fermenter (0) as claimed in any of the preceding claims, wherein before or after finishing the fermenter shell (3) a heating system (32) projecting into the interior of the fermenter cage is arranged through rotation of the fermenter cage (1) about the longitudinal axis (L) .

5. The construction method of a fermenter (0) as claimed in any of the preceding claims, wherein following the finishing of the fermenter shell (3) an agitator unit (33) is inserted into the interior of the fermenter cage and is fastened there through rotation of the fermenter cage (1) about the longitudinal axis (L).

6. The construction method of a fermenter (0) as claimed in claim 5, wherein the agitator unit (33) is introduced into the fermenter cage (1) from an input side (E) linearly in the direction of the longitudinal axis (L).

7. The construction method of a fermenter (0) as claimed in any of the preceding claims, wherein directly following the finishing of the fermenter shell (3) a wall structure (31) on the output side is permanently welded gas-tight onto the last cage ring (10x) and a wall structure (34) on the input side is permanently welded gas-tight onto the first cage ring (10).

8. The construction method of a fermenter (0) as claimed in any of the preceding claims, wherein prior to positioning, the cage rings (10 to 10x) are assembled together from a plurality of ring segments (100, 100', 100") and connected to one another.

9. The construction method of a fermenter (0) as claimed in claim 8, wherein the ring segments (100, 100', 100") have at their ends flange plates, which are detachably connected to one another by means of retaining ring bolts.

10. The construction method of a fermenter (0) as claimed in claim 8 or 9, wherein the cage rings (10 to 10x) are assembled from three similar-sized ring segments (100, 100', 100").

11. The construction method of a fermenter (0) as claimed in any of the preceding claims, wherein the cage rings (10 to 10x) are formed from double-T supports, which can be bent cold.

12. The construction method of a fermenter (0) as claimed in any of the preceding claims, wherein prior to positioning the cage rings (10 to 10x), the plurality of cage-retaining devices (2) are arranged with rolling devices (200) in one row.

13. The construction method of a fermenter (0) as claimed in any of the preceding claims, wherein the shell plates (30 to 30n) do not exceed a thickness of eight millimeters.

14. The construction method of a fermenter (0) as claimed in claim 1, wherein the fermenter cage (1) is rotated into a welding position so that a longitudinal welding seam (S) is welded between adjacent rows of shell plates (30 to 30n) in a position facing the cage-retaining devices (2).

15. The construction method of a fermenter (0) as claimed in claim 14, wherein the welding of the rows of shell plates (30 to 30n) is carried out by a welding robot.

16. The construction method of a fermenter (0) as claimed in claim 14, wherein several transverse slits (Q) are arranged in the shell plates (30 to 30n) so that the transverse slits (Q) each come to lie on the interior faces of the cage rings (10) when the shell plates (30 to 30n) are inserted in the fermenter cage (1).

17. The construction method of a fermenter (0) as claimed in claim 14, wherein after fixing the shell plates (30 to 30n) on the cage rings (10 to 10x), the longitudinal welding seams (S) between directly adjacent rows of shell plates (30 to 30n) are welded gas-tight.

18. The construction method of a fermenter (0) as claimed in any of the preceding claims, wherein after closing the fermenter shell (3), an insulation shell (4) is attached and fastened on the fermenter shell (3) through rotation of the fermenter cage (1) about the longitudinal axis (L).

19. The construction method of a fermenter (0) as claimed in claim 18, wherein the insulation shell (4) is formed by a plurality of rigid or flexible insulation elements (40) or by a foam.

## Revendications

1. Méthode de construction d'un fermentateur (0) avec une cage de fermentateur (1) et une enveloppe de fermentateur (3) en métal, pour une installation de biogaz, **caractérisée en ce que** la cage de fermentateur (1) est formée par une multitude d'anneaux de cage (10 à 10x), chaque anneau de cage (10 à 10x) étant supporté par au moins un dispositif de support de cage (2), comprenant au moins un dispositif de roulage (200), de telle sorte que la cage de fermentateur (1) entière est positionnée de façon rotative autour d'un axe longitudinal (L), et une multitude de traverses de raccordement (11) s'étendant entre les anneaux de cage (10, 10') directement adjacents est fixée, la cage de fermentateur (1) étant recouverte avec une multitude de rangées de plaques d'enveloppe (30 à 30n) par une rotation autour de l'axe longitudinal (L), et les plaques d'enveloppe (30 à 30n) étant fixées de façon permanente à la multitude d'anneaux de cage (10 à 10x) et l'enveloppe de fermentateur (3) fermée de façon étanche au gaz étant ainsi formée pas à pas.

2. Méthode de construction d'un fermentateur selon la revendication 1, **caractérisée par** les étapes :
I) introduire une première rangée de plaques d'enveloppe (30) dans la cage de fermentateur (1) parallèlement à l'axe longitudinal (L),
II) plier les premières plaques d'enveloppe (30) correspondant au tracé des surfaces intérieures des anneaux de cage (10),
III) fixation de façon permanente des premières plaques d'enveloppe (30) aux surfaces intérieures des anneaux de cage (10 à 10x) faisant face vers l'intérieur de la cage de fermentateur, et ensuite
IV) rotation de la cage de fermentateur (1) autour de l'axe longitudinal (L) de telle sorte qu'une rangée adjacente d'autres plaques d'enveloppe (30' à 30n) peut être placée dans la cage de fermentateur (1) à côté des plaques d'enveloppe (30 à 30n-1) disposées précédemment et
V) répétition des étapes I) à IV) jusqu'à ce que la nième rangée de plaques d'enveloppe (30 à 30n) soit fixée et la cage de fermentateur (1) soit recouverte complètement de façon étanche au gaz par l'enveloppe de fermentateur (3), comprenant n rangées de plaques d'enveloppe (30 à 30n).

3. Méthode de construction d'un fermentateur selon la revendication 2, la fabrication de la cage de fermentateur (1) étant réalisée par
a) placement verticalement de plusieurs anneaux de cage (10 à 10x) sur des dispositifs de support de cage (2) avec le dispositif de roulage (200) de telle sorte que les anneaux de cage (10 à 10x) soient disposés parallèlement l'un à l'autre et soient reliés fonctionnellement avec le au moins un dispositif de roulage (200) de telle sorte qu'un positionnement de façon rotative de chaque anneau de cage (10 à 10x) soit atteint et
b) fixation de plusieurs traverses de raccordement (11) entre des anneaux de cage (10 à 10x) adjacents parallèlement à l'axe longitudinal (L) de la cage de fermentateur (1) résultante et répétition des étapes a) et b) jusqu'à ce que la longueur de cage de fermentateur désirée soit atteinte.

4. Méthode de construction d'un fermentateur (0) selon l'une quelconque des revendications précédentes, un système de chauffage (32) qui dépasse à l'intérieur de la cage de fermentateur étant disposé, avant ou après l'achèvement de l'enveloppe de fermentateur (3), par rotation de la cage de fermentateur (1) autour de l'axe longitudinal (L).

5. Méthode de construction d'un fermentateur (0) selon l'une quelconque des revendications précédentes, un agitateur (33) étant inséré, à la suite de l'achèvement de l'enveloppe de fermentateur (3), à l'intérieur de la cage de fermentateur et fixé dedans par rotation de la cage de fermentateur (1) autour de l'axe longitudinal (L).

6. Méthode de construction d'un fermentateur (0) selon la revendication 5, l'agitateur (33) étant introduit dans la cage de fermentateur (1) d'un côté d'entrée (E) linéairement dans la direction de l'axe longitudinal (L).

7. Méthode de construction d'un fermentateur (0) selon l'une quelconque des revendications précédentes, directement à la suite de l'achèvement de l'enveloppe de fermentateur (3), une structure de paroi (31) sur le côté de sortie étant soudée de façon permanente de façon étanche au gaz au dernier anneau de cage (10x) et une structure de paroi (34) sur le côté d'entrée étant soudée de façon permanente de façon étanche au gaz au premier anneau de cage (10).

8. Méthode de construction d'un fermentateur (0) selon l'une quelconque des revendications précédentes, les anneaux de cage (10 à 10x) avant le positionnement étant assemblés ensemble à partir d'une multitude de segments d'anneau (100, 100', 100") et connectés l'un à l'autre.

9. Méthode de construction d'un fermentateur (0) selon la revendication 8, les segments d'anneau (100, 100', 100") ayant à leurs extrémités des plaques de bridage, qui sont reliées de façon détachable limitée l'une à l'autre au moyen de goujons à bague de serrage.

10. Méthode de construction d'un fermentateur (0) selon la revendication 8 ou 9, les anneaux de cage (10 à 10x) étant assemblés à partir de trois segments d'anneau (100, 100', 100") de taille similaire.

11. Méthode de construction d'un fermentateur (0) selon l'une quelconque des revendications précédentes, les anneaux de cage (10 à 10x) étant formés à partir de supports en double-T, qui peuvent être pliés à froid.

12. Méthode de construction d'un fermentateur (0) selon l'une quelconque des revendications précédentes, avant le positionnement des anneaux de cage (10 à 10x), la multitude des dispositifs de support de cage (2) avec des dispositifs de roulage (200) étant disposés en une rangée.

13. Méthode de construction d'un fermentateur (0) selon l'une quelconque des revendications précédentes, les plaques d'enveloppe (30 à 30n) n'excédant une épaisseur de huit millimètres.

14. Méthode de construction d'un fermentateur (0) selon la revendication 1, la cage de fermentateur (1) étant tournée dans une position de soudure de telle sorte qu'un joint de soudure longitudinal (S) est soudé entre des rangées adjacentes de plaques d'enveloppe (30 à 30n) dans une position faisant face aux dispositifs de support de cage (2).

15. Méthode de construction d'un fermentateur (0) selon la revendication 14, la soudure des rangées de plaques d'enveloppe (30 à 30n) étant réalisée par un robot de soudure.

16. Méthode de construction d'un fermentateur (0) selon la revendication 14, plusieurs fentes transversales (Q) étant disposées dans les plaques d'enveloppe (30 à 30n) de telle sorte que les fentes transversales (Q) chacune viennent se situer sur les surfaces intérieures des anneaux de cage (10) quand les plaques d'enveloppe (30 à 30n) sont insérées dans la cage de fermentateur (1).

17. Méthode de construction d'un fermentateur (0) selon la revendication 14, après la fixation des plaques d'enveloppe (30 à 30n) sur les anneaux de cage (10 à 10x), les joints de soudure longitudinaux (S) entre des rangées directement adjacentes de plaques d'enveloppe (30 à 30n) étant soudées de façon étanche au gaz.

18. Méthode de construction d'un fermentateur (0) selon l'une quelconque des revendications précédentes, après la fermeture de l'enveloppe de fermentateur (3), une enveloppe d'isolation (4) étant attachée et fixée sur l'enveloppe de fermentateur (3) par rotation de la cage de fermentateur (1) autour de l'axe longitudinal (L).

19. Méthode de construction d'un fermentateur (0) selon la revendication 18, l'enveloppe d'isolation (4) étant formée par une multitude d'éléments d'isolation (40) rigides ou flexibles ou par une mousse.
